# EUROPEAN PATENT APPLICATION

(11) **EP 4 410 321 A1**
(43) Date of publication of application: **07.08.2024**
(21) Application number: 23856574.1
(22) Date of filing: 21.08.2023
(51) Int. Cl.: A61L 24/00, A61L 24/02

(54) **VASCULAR EMBOLIC AGENT, METHOD FOR PREPARING SAME AND USE THEREOF**

(30) Priority: 24.08.2022 CN 202211019192
(71) Applicant: The Fifth Affiliated Hospital Sun Yat-Sen University, Zhuhai, Guangdong 519000 (CN)
(72) Inventor: SHAN, Hong, Zhuhai, Guangdong 519000 (CN); PENG, Xin, Zhuhai, Guangdong 519000 (CN); LIU, Menghui, Zhuhai, Guangdong 519000 (CN)
(74) Representative: Gill Jennings & Every LLP
(86) International application number: PCT/CN2023/114026
(87) International publication number: WO 2024/041484

(57) **Abstract**

Provided are a vascular embolic agent, a method for preparing same and use thereof. The method for preparing the vascular embolic agent comprises the following steps: (1) dissolving a 1,2-dithiolane compound, a polyphenol compound and an alkaloid in an organic solvent A to obtain a mixed solution A; (2) sealing the mixed solution A, and reacting same in an environment of 70°C or above for 5-12 hours to obtain a mixed solution B; and (3) cooling the mixed solution B to room temperature, and adding an organic solvent B into the cooled mixed solution B for dilution to obtain the vascular embolic agent. The vascular embolic agent prepared by means of the method has outstanding biocompatibility, stable mechanical performance, excellent intravascular properties, is easily delivered to microvessels and complex-shaped target blood vessels, does not adhere to blood vessels, and can be developed without imaging artifacts.

## Description

The present application claims the priority of Chinese Patent Application No. 202211019192.1, filed with the China National Intellectual Property Administration on August 24, 2022, and titled with "vascular embolic agent, method for preparing same and use thereof, which is hereby incorporated by reference in its entirety.

### FIELD

The present disclosure relates to the technical field of vascular embolic agents, and particularly relates to a vascular embolic agent, a method for preparing same and use thereof.

### BACKGROUND

Vascular embolotherapy has advantages such as minimal invasiveness, high repeatability and rapid effect, and has thus become an effective means for the clinical treatment of vascular lesions and neoplastic lesions, particularly hemorrhagic lesions, vascular malformations, aneurysms, and intermediate-stage primary liver cancer according to the Barcelona Clinic Liver Cancer (BCLC) staging system. Based on their physical properties, embolic materials that are used in clinical practice can be divided into solid and liquid embolic agents. Solid embolic materials mainly include spring coils, gelatin sponge particles and polyvinyl alcohol microspheres. Liquid embolic materials mainly include N-butyl-cyanoacrylate (nBCA), Onyx with the main components of ethylene-vinyl alcohol copolymer and dimethyl sulfoxide and iodide oil.

Spring coils tend to cause vessel rupture during embolization and may fail to reach the target site due to over-tortuous blood vessels. Gelatin sponge particles are prone to degradation, resulting in a recanalization of blood vessels after occlusion. Both polyvinyl alcohol microspheres and gelatin sponge particles are prone to agglomeration, making it difficult to reach the microvasculature. nBCA undergoes rapid polymerization upon contact with blood to create an embolus. However, the reaction product has poor migration and tends to adhere to the catheters, posing a great risk to the patient. Onyx is non-degradable and easily causes local chronic inflammation and/or rejection by the body. Iodide oil has strong fluidity, bringing easy vessel recanalization, poor embolization, resulting in ineffective loading and sustained drug release. In addition, most embolic agents and developers cannot be tightly cross-linked, leading to the easy separation of contrast agents from embolic agents during surgery or after surgery. Therefore, it is difficult to effectively monitor the location of the embolic agent, resulting in incomplete embolization or ectopic embolization.

### SUMMARY

The present disclosure aims at to overcome the above deficiencies in the prior art and to provide a vascular embolic agent, a method for preparing same and use thereof. The vascular embolic agent has stable mechanical properties, excellent biocompatibility, and good migration in the blood vessel, is easily delivered to microvessels and complex-shaped target blood vessels, does not adhere to blood vessels, and can effectively load drugs. The vascular embolic agent is tightly cross-linked with the contrast agent and can be developed without imaging artifacts.

The technical solution used in the present disclosure to achieve the above purpose is as follows.

A method for preparing a vascular embolic agent, comprises the following steps:
Step (1), dissolving a 1,2-dithiolane compound, a polyphenol compound and an alkaloid in an organic solvent A to obtain a mixed solution A;
Step (2), sealing the mixed solution A, and conducting a reaction in an environment of 70°C or above for 5-12 hours to obtain a mixed solution B; and
Step (3), cooling the mixed solution B to room temperature, and adding an organic solvent B into the mixed solution B for dilution to obtain the vascular embolic agent.

A 1,2-dithiolane compound may be grafted onto the phenyl ring of a polyphenol compound via Michael addition reaction to form C-S bond between these two compounds. The carboxyl group and the like of a 1,2-dithiolane compound may form a hydrogen bond with the phenolic hydroxyl group, carboxyl group or amino group of a polyphenol compound. In addition, the alkaloid can neutralize the excess hydrogen ions in the system to reduce the cytotoxicity of the resulting product and avoid irritation of the blood vessel wall by the same, and also increase the cross-linking density of the resulting gel through the hydrogen bonding with the 1,2-dithiolane compound and polyphenol compound to improve the embolization effect. After being injected into physiological fluids (such as blood) as well as aqueous solutions, the vascular embolic agent can rapidly form a gel.

Preferably, the organic solvent A and organic solvent B are selected from at least one of dimethyl sulfoxide (DMSO) and ethanol, respectively. The organic solvent A and organic solvent B are the same or different. Further preferably, both the organic solvent A and organic solvent B are dimethyl sulfoxide. Dimethyl sulfoxide is less irritating to blood vessels compared with other organic solvents.

Preferably, in step (1), the mass ratio of the 1,2-dithiolane compound, the polyphenol compound, and the alkaloid is (0.05-1):(0.0001-1):(0.01-1), the mass ratio of the organic solvent A to the mixed solution A is (2-4):10; and in step (3), the mass ratio of the organic solvent B to the vascular embolic agent is (1-5.8):10. By optimizing the ratios of these components as described above, it can be ensured that the reaction can be carried out adequately and that the prepared vascular embolic agent has a good embolic effect.

Preferably, in step (1), the mass ratio of the 1,2-dithiolane compound, the polyphenol compound, and the alkaloid is 0.6:0.05:0.3. In the case of satisfying the above ratios, the prepared vascular embolic agent can be coagulated quickly, does not easily adhere to catheter, has excellent operability, and has good mechanical properties, and has a good occlusion effect on the blood vessel.

Preferably, in step (2), the mixed solution A is subjected to reaction at 90°C for 10 hours. The reaction product prepared under this condition has stable mechanical properties, low viscosity and a suitable gelling time, and the reaction is high efficient under this condition.

Preferably, the 1,2-dithiolane compound is at least one of lipoic acid, asparagusic acid, dithiolopyrrolone antibiotics, and kottamide E. The polyphenol compound is at least one of tannic acid, gallic acid, caffeic acid, catechol, dopamine, polydopamine, resveratrol, quercetin, curcumin, chlorogenic acid, isoflavone, anthocyanin, cocoa polyphenol, limocitrin, catechin, and rutin. The alkaloid is at least one of tromethamine, ephedrine, leonurine and cinchona.

Further preferably, the 1,2-dithiolane compound is lipoic acid, the polyphenol compound is tannic acid, and the alkaloid is tromethamine. These compositions have good biocompatibility, are highly reactive and have high economic benefits.

Preferably, in step (3), after dilution, a contrast agent is added in an amount of 10 wt%-80wt% by mass of the vascular embolic agent, and the contrast agent is a liquid metal having a melting point of less than or equal to 35°C or tantalum microparticles. The addition of the contrast agent may render the vascular embolic agent which is X-ray radiopaque, being advantageous for detecting the position of the vascular embolic agent during and after surgery.

Further preferably, the contrast agent is added in an amount of 30wt% of the vascular embolic agent, and the contrast agent is a gallium-indium alloy. The contrast agent can be firmly and stably combined with the product obtained by the reaction of the 1,2-dithiolane compound and the polyphenol compound by electrostatic interaction or chelation, and will not detach from the embolic agent during use or prolonged placement, thereby enabling precise positioning of the vascular embolic agent during and after the surgery.

In addition, the present disclosure further discloses a vascular embolic agent prepared by the above-mentioned method and the use of the vascular embolic agent in the manufacture of a medicament carrier. And the present disclosure further discloses the use of the vascular embolic agent in the treatment of hemorrhagic lesions, aneurysms, and arteriovenous malformations in arteries and veins of various organs in the human body, and the use of the vascular embolic agent loaded with a chemotherapeutic drug, targeting drug, or an immune inhibitor in the chemoembolization treatment for tumors.

Compared to the prior art, the present disclosure has the following beneficial effects.
(1) The vascular embolic agent of the present disclosure, which is prepared with a 1,2-dithiolane compound, a polyphenol compound, and an alkaloid as the main raw materials, has a suitable solidification time and moderate viscosity after being injected into physiological fluids (such as blood) or an aqueous solution, and has a better operability. The resulting gel does not adhere easily to catheter, allowing easy removal of catheter after embolization surgery.
(2) After solidification, the vascular embolic agent of the present disclosure has good mechanical strength, embolization pressure and excellent embolization effect.
(3) The raw materials selected for the preparation in the present disclosure have good biocompatibility, and the prepared vascular embolic agent has excellent migration, allowing delivery to microvessels and complex-shaped target vessels.
(4) In the present disclosure, liquid metal or tantalum microparticles, which can combine with the product obtained by the reaction of the 1,2-dithiopentane compound and the polyphenol compound by electrostatic interaction or chelation, are used as contrast agent, which facilitates monitoring of the location of the embolic agent during surgery, reduces imaging artifact, and also facilitates monitoring post-interventional therapeutic efficacy.
(5) The vascular embolic agent of the present disclosure may be useful in the treatment of hemorrhagic lesions, aneurysms and arteriovenous malformations in various organs of the human body, and may be useful in arterial embolization to block the blood supply to benign or malignant tumors, and may be useful in chemoembolization for malignant tumors; wherein the chemoembolization comprises effective loading of a sustained release drug (such as at least one of adriamycin, cisplatin, carboplatin, lobaplatin, raltitrexed, gemcitabine, 5- fluorouracil, irinotecan, bleomycin and vinpocetine); a targeting drug (such as at least one of sorafenib, lenvatinib, regorafenib, cabozantinib, apatinib, and anrotinib); and an immune inhibitor (such as at least one of atezolizumab, keytruda, nivolumab, karelizumab, and cindilizumab).

### BRIEF DESCRIPTION OF DRAWINGS

Figure 1 is a schematic diagram of preparation process of a vascular embolic agent using a 1,2-dithiolane compound and a polyphenol compound as the main raw materials.
Figure 2 is a schematic diagram of preparation process of a vascular embolic agent using lipoic acid and tannic acid as the main raw materials.
Figure 3 shows the Raman spectra of lipoic acid and poly (lipoic acid-tannic acid).
Figure 4 is an overview for the injection force test of embolic agents, wherein A is a schematic diagram of the testing, B shows the test results of different embolic agents in 1.7F microcatheters, C shows the specifications of different catheters, and D shows the results of the injection force test of the PLA-TA-Tro-Ga embolic agent in different catheters.
Figure 5 is an overview for in-vitro embolization force test, wherein A is a schematic diagram of the testing, and B shows the test results of different embolic agents.
Figure 6 shows the results of biocompatibility test, wherein A shows the hemolysis photographs in different experimental groups, B shows results of hemolysis rate test, C shows the results of live/dead cell staining for L929 cells after co-incubation with different embolic agents, and D is a diagram of cell survival rate test.
Figure 7 shows an in-vitro drug release profile, wherein A-C are release profiles loaded with adriamycin, sorafenib, and atilizumab, respectively.
Figure 8 is the diagram of in-vitro and in-vivo X-ray imaging, wherein A is an X-ray image of the syringe loaded with the vascular embolic agent, B is an X-ray image of a rabbit ear artery and branch blood vessels containing the vascular embolic agent, and C is an X-ray image of a renal artery and branch blood vessels containing the vascular embolic agent.
Figure 9 is a comparison showing the rabbit renal artery and renal parenchyma before and after embolization. A is the optical image before and after rabbit renal artery embolization, B is the color Doppler ultrasound imaging before and after rabbit renal artery embolization, and C is the two-dimensional gray scale image before and after renal parenchyma embolization. and D is the color Doppler ultrasound imaging before and after renal parenchyma embolization.
Figure 10 is a comparison showing the rabbit femoral artery before and after embolization, wherein A is the optical image of the rabbit femoral artery before embolization, B is the ultrasonic color Doppler image of the rabbit femoral artery before embolization, C is the optical image of rabbit femoral artery after embolization, and D is the ultrasonic color Doppler image of the rabbit femoral artery after embolization.
Figure 11 is a schematic diagram of the preparation process of the vascular embolic agent in example 3.
Figure 12 is a schematic diagram of the preparation process of the vascular embolic agent in example 4.
Figure 13 is a schematic diagram of the preparation process of the vascular embolic agent in example 5.
Figure 14 is a schematic diagram of the preparation process of the vascular embolic agent in example 6.
Figure 15 is a schematic diagram of the preparation process of the vascular embolic agent in example 7.
Figure 16 is a schematic diagram of the preparation process of the vascular embolic agent in example 8.
Figure 17 is a schematic diagram of the preparation process of the vascular embolic agent in example 9.
Figure 18 is a schematic diagram of the preparation process of the vascular embolic agent in example 10.
Figure 19 is a schematic diagram of the preparation process of the vascular embolic agent in example 11.
Figure 20 is a schematic diagram of the preparation process of the vascular embolic agent in example 12.
Figure 21 is a schematic diagram of the preparation process of the vascular embolic agent in example 13.
Figure 22 is a schematic diagram of the preparation process of the vascular embolic agent in example 14.
Figure 23 is a schematic diagram of the preparation process of the vascular embolic agent in example 15.
Figure 24 is a schematic diagram of the preparation process of the vascular embolic agent in example 16.
Figure 25 is a schematic diagram of the preparation process of the vascular embolic agent in example 17.

### DETAILED DESCRIPTION

In order to better illustrate the purpose, technical solutions and advantages of the present disclosure, the present disclosure will be further described below in conjunction with specific examples and accompanying drawings.

### Examples

### Example 1

In an example of the present disclosure, a method for preparing the vascular embolic agent of the present disclosure is as follows. The mass ratios of the components are shown in Table 1.
(1) Lipoic acid, tannic acid and tromethamine powder were dissolved in DMSO in different mass ratios to obtain a mixed solution A;
(2) the mixed solution A was sealed and placed at 90°C for reaction for 10 hours, a mixed solution B was obtained; and
(3) the mixed solution B was cooled to room temperature and DMSO was added for dilution to obtain the vascular embolic agent, recorded as PLA-TA-Tro (DMSO).

The PLA-TA-Tro (DMSO) was mixed with simulated physiological fluid to form a PLA-TA-Tro hydrogel, recorded as PLA-TA-Tro (Gel).

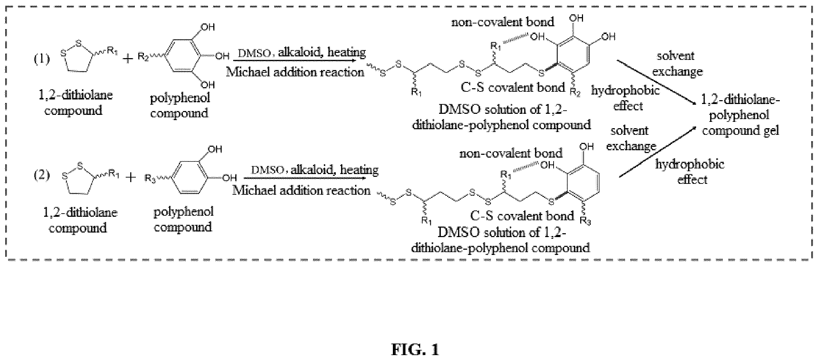

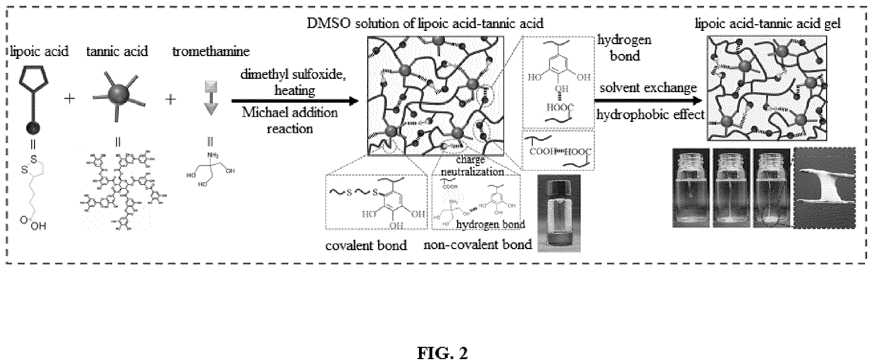

Figure 1 is a schematic diagram showing the route for preparing a vascular embolic agent using a 1,2-dithiolane compound and a polyphenol compound as the main raw materials. Figure 2 is a schematic diagram of the route for preparing a vascular embolic agent using lipoic acid and tannic acid as the main raw materials, showing that a Michael addition reaction between lipoic acid and tannic acid produced a compound containing both a C-S covalent bond and a hydrogen bond, and this compound, together with an alkaloid and DMSO, constituted the vascular embolic agent of the present disclosure. The vascular embolic agent was injected into physiological fluids (such as blood) or water, and PLA-TA-Tro (DMSO) can form a dense three-dimensional cross-linked network driven by hydrophobic effect and the like, thus PLA-TA-Tro (Gel) is formed.

The solidification time of different PLA-TA-Tro (DMSO) prepared with different raw material ratios, pH, viscosity, and storage modulus of PLA-TA-Tro (Gel) were tested, and the results are shown in Table 1.

**Table 1**

| Item | lipoic acid (g) | tannic acid (g) | trometh amine (g) | DMSO (g) | | pH | solidif icatio n-time (min) | viscosity (mPa·s) | storage modulus (kPa) |
|---|---|---|---|---|---|---|---|---|---|
| | | | | Step (1) | Step (3) | | | | |
| Sample 1 | 1.2 | 0.05 | 0 | 0.5 | 1.0 | 3.8 | 5.0 | 320 | 490 |
| Sample 2 | 1.2 | 0.05 | 0.2 | 0.5 | 1.0 | 5.0 | 5.5 | 310 | 481 |
| Sample 3 | 1.2 | 0.05 | 0.4 | 0.5 | 1.0 | 5.6 | 6.3 | 311 | 479 |
| Sample 4 | 1.2 | 0.05 | 0.6 | 0.5 | 1.0 | 6.3 | 7.0 | 324 | 460 |
| Sample 5 | 1.2 | 0.05 | 0.8 | 0.5 | 1.0 | 7.1 | - | - | - |
| Sample 6 | 1.2 | 0.10 | 0 | 0.5 | 1.0 | 3.6 | 4.4 | 335 | 520 |
| Sample 7 | 1.2 | 0.10 | 0.2 | 0.5 | 1.0 | 4.9 | 4.9 | 356 | 516 |
| Sample 8 | 1.2 | 0.10 | 0.4 | 0.5 | 1.0 | 5.4 | 7.1 | 340 | 514 |
| Sample 9 | 1.2 | 0.10 | 0.6 | 0.5 | 1.0 | 6.2 | 8.0 | 320 | 509 |
| Sample 10 | 1.2 | 0.10 | 0.8 | 0.5 | 1.0 | 7.2 | - | - | - |
| Sample 11 | 1.2 | 0.15 | 0 | 0.5 | 1.0 | 3.4 | 3.4 | 410 | 520 |
| Sample 12 | 1.2 | 0.15 | 0.2 | 0.5 | 1.0 | 4.6 | 4.3 | 401 | 516 |
| Sample 13 | 1.2 | 0.15 | 0.4 | 0.5 | 1.0 | 5.2 | 5.1 | 399 | 514 |
| Sample 14 | 1.2 | 0.15 | 0.6 | 0.5 | 1.0 | 6.1 | 6.2 | 388 | 509 |
| Sample 15 | 1.2 | 0.15 | 0.8 | 0.5 | 1.0 | 7.1 | - | - | - |
| Sample 16 | 1.2 | 0.20 | 0 | 0.5 | 1.0 | 3.0 | 3.3 | 450 | 560 |
| Sample 17 | 1.2 | 0.20 | 0.2 | 0.5 | 1.0 | 4.5 | 4.1 | 446 | 546 |
| Sample 18 | 1.2 | 0.20 | 0.4 | 0.5 | 1.0 | 5.2 | 4.8 | 441 | 544 |
| Sample 19 | 1.2 | 0.20 | 0.6 | 0.5 | 1.0 | 5.4 | 6.6 | 440 | 536 |
| Sample 20 | 1.2 | 0.20 | 0.8 | 0.5 | 1.0 | 5.5 | - | - | - |
| Sample 21 | 1.2 | 0.10 | 0.6 | 0.5 | 1.5 | 6.2 | 7.9 | 200 | 489 |
| Sample 22 | 1.2 | 0.10 | 0.6 | 0.5 | 2.0 | 6.3 | 9.8 | 100 | 470 |
| Sample 23 | 1.2 | 0.10 | 0.6 | 0.5 | 2.5 | 6.1 | 10.2 | 89 | 355 |
| Sample 24 | 1.2 | 0.10 | 0.6 | 0.5 | 3.0 | 6.2 | 12.5 | 78 | 236 |
| Sample 25 | 1.2 | 0.10 | 0.6 | 0.5 | 3.5 | 6.3 | - | - | - |

Note: In samples 5, 10, 15 and 20, due to the high concentration of tromethamine, a strong electrostatic interaction was formed between it and the carboxyl group on the poly(lipoic acid), preventing the formation of hydrogen bonds between the polymers, and resulting in the inability to form a gel, which is indicated by "-". The concentration of DMSO in sample 25 was too high, thereby significantly reducing the concentration and interaction force of the polymers, and resulting in the inability to form a gel by hydrogen bond cross-linking, which is indicated by "-".

As can be seen from Table 1, Sample 22 has the optimal comprehensive performance, PLA-TA-Tro (DMSO) has a suitable solidification time, PLA-TA-Tro (Gel) has a suitable viscosity. The vascular embolic agent has a good operability and usability, and does not easily adhere to the catheters, allowing easy removal of the catheter after embolization.

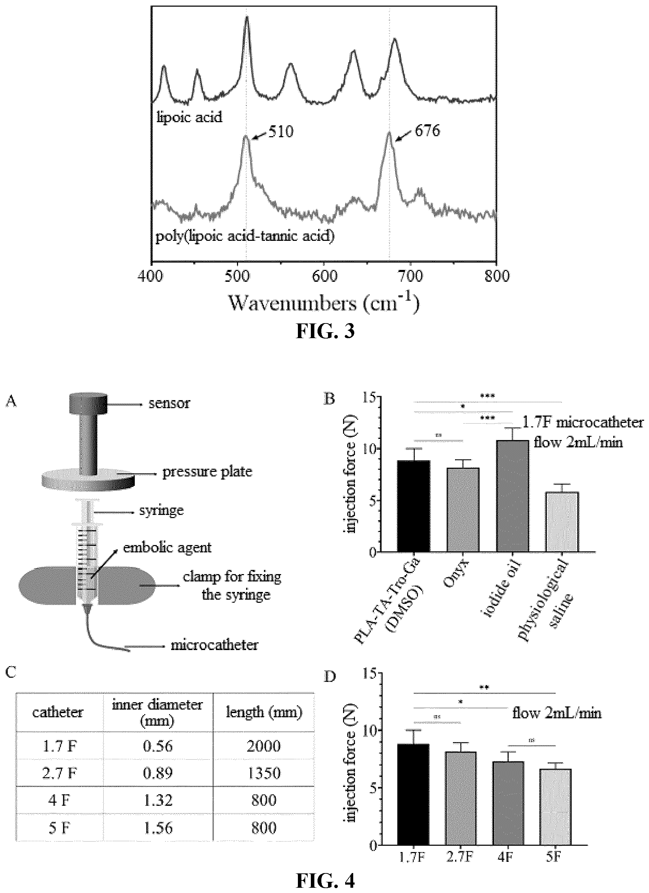

Figure 3 shows the Raman spectra of the lipoic acid monomer and the vascular embolic agent (lipoic acid and poly (lipoic acid-tannic acid)) of sample 22, wherein the peak at 510 cm⁻¹ indicates the S-S bond and the peak at 676 cm⁻¹ indicates the C-S bond. The results show the successful grafting of the 1,2-dithiolane compound onto the polyphenol compound.

### Example 2

In an example of a method for preparing the vascular embolic agent of the present disclosure, a method for preparing the vascular embolic agent is as follows. The vascular embolic agent PLA-TA-Tro (DMSO) of Sample 22 in Example 1 was mixed with gallium-indium-tin alloy in different ratios to obtain the vascular embolic agent PLA-TA-Tro-Ga (DMSO), the ratios of the two components are shown in Table 2. The PLA-TA-Tro-Ga (DMSO) was injected to a simulated physiological solution (8.035g sodium chloride, 0.355g sodium bicarbonate, 0.225g potassium chloride, 0.231g dipotassium hydrogen phosphate, 0.311g magnesium chloride hexahydrate, 39mL 1M hydrochloric acid, 0.292g calcium chloride, 0.072g sodium sulfate and 6.118g tromethamine in 1L of deionized water) to obtain a PLA-TA-Tro-Ga hydrogel, recorded as PLA-TA-Tro-Ga (Gel).

The solidification time of different PLA-TA-Ga (DMSO) prepared with different raw material ratios, the viscosity, storage modulus and CT values of PLA-TA-Ga (Gel) were tested, and the results are shown in Table 2.

**Table 2**

| Item | PLA-TA-Tro (DMSO) (g) | gallium-indium-tin alloy (g) | solidification time (minute) | viscosity (mPa·s) | storage modulus (kPa) | CT value (Hu) |
|---|---|---|---|---|---|---|
| Sample 26 | 10 | 0 | 11 | 50 | 430 | 20 |
| Sample 27 | 9 | 1 | 10.5 | 61 | 445 | 300 |
| Sample 28 | 8 | 2 | 10 | 68 | 450 | 500 |
| Sample 29 | 7 | 3 | 9 | 75 | 465 | 1100 |
| Sample 30 | 6 | 4 | 18 | 90 | 320 | 1200 |
| Sample 31 | 5 | 5 | 23 | 79 | 300 | 1350 |
| Sample 32 | 4 | 6 | 26 | 67 | 178 | 1500 |
| Sample 33 | 3 | 7 | 31 | 55 | 105 | 1550 |
| Sample 34 | 2 | 8 | 40 | 40 | 50 | 1690 |
| Sample 35 | 1 | 9 | - | - | - | 2000 |

Note: The concentration of liquid metal in Sample 35 was too high, thereby significantly reducing the concentration and interaction force of the polymers, resulting in the inability to form a gel by hydrogen bonding cross-linking, which is indicated by " ".

As can be seen from Table 2, the optimal comprehensive performance was achieved in the case where the contrast agent was added in an amount of 30wt% by mass of the vascular embolic agent PLA-TA-Tro-Ga (DMSO). The solidification time of PLA-TA-Tro-Ga (DMSO) was 9 minutes, and the resulting PLA-TA-Tro-Ga (Gel) has a moderate viscosity, a very high storage modulus, and an excellent embolization effect. Also, the gel has a CT value of over 1000 Hu and it is easy to track it during the treatment.

The PLA-TA-Tro-Ga (DMSO) used in the following property tests was the vascular embolic agent PLA-TA-Tro-Ga (DMSO) of sample 32.

Injection force test of the embolic agents was carried out. A 1mL luer-lock syringe was placed on a lab-made test apparatus as shown in Figure 4A, with the upper end of the syringe in contact with the upper pressure plate, and the lower end fixed perpendicularly to the pressure plate by a clamp and connected to catheters of different sizes. Specifically, 1) 1mL of PLA-TA-Tro-Ga (DMSO), Onyx, iodide oil, and physiological saline, were added into the syringe, a 1.7F microcatheter was connected to the lower end of the syringe, and then the syringe was uniformly pressed with the upper pressure plate at a flow rate of 2mL/min, while the pressure change of the upper pressure plate was recorded. The result is shown Figure 4B, in which the injection force of iodide oil, PLA-TA-Tro-Ga (DMSO), Onyx, and physiological saline were in order high to low, indicating that the injection of PLA-TA-Tro-Ga (DMSO) is relatively easy. 2) 1mL of PLA-TA-Tro-Ga (DMSO) was added into the syringe, and catheters with different sizes (Figure 4C) were connected to the lower end of the syringe, then the syringe was uniformly pressed with the upper pressure plate at a flow rate of 2mL/min, while the pressure change of the upper pressure plate was recorded, and the result was shown in Figure 4D, in which the injection force decreased with the increase of the diameter of the microcatheter. In conclusion, PLA-TA-Tro-Ga (DMSO) was easily injected through the catheter and had excellent usability.

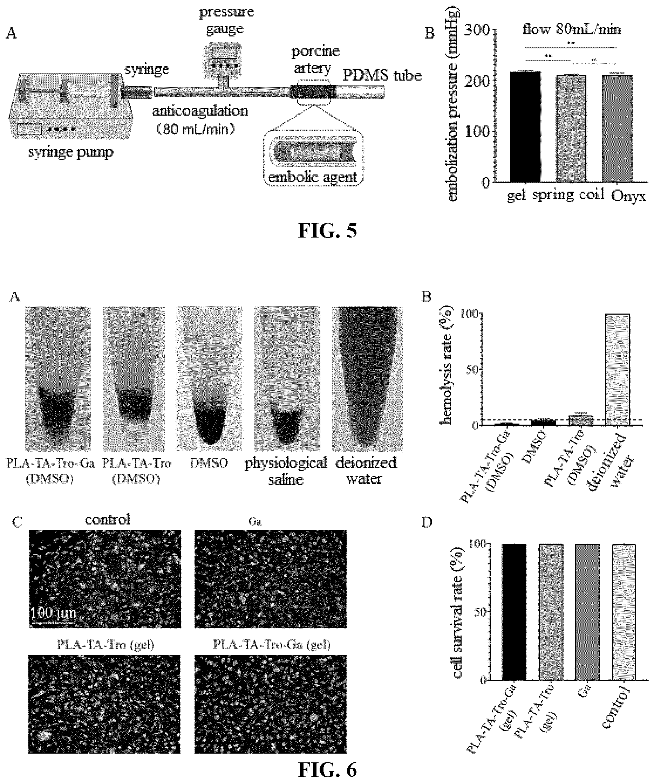

Embolization test is carried out in vitro. The syringe pump, syringe, pressure gauge, porcine artery (inner diameter: 6mm), and polydimethylsiloxane (PDMS) tube (inner diameter: 6mm) were connected according to Figure 5A, and the whole tube was filled with anticoagulated blood at 37°C, then the porcine artery and PDMS tube were immersed in the anticoagulated blood at 37°C. PLA-TA-Tro-Ga (DMSO) (1mL), Onyx (1mL), and a spring coil (6 mm*6mm) were then injected separately into the porcine artery through a 5F microcatheter (inner diameter: 1.7mm). After removal of tube (the vascular embolic agent had formed a gel), the anticoagulated blood was pushed at a flow rate of 80mL/min until the vascular embolic gel PLA-TA-Tro-Ga (Gel) was ejected from blood vessels, while the change in systemic pressure was recorded with a pressure gauge, with the maximum value recorded as the embolic pressure of the embolic agent. As can be seen in Figure 5B, PLA-TA-Tro-Ga (Gel) has a higher embolic pressure compared to spring coils and Onyx, which may favor embolization of arterial vessels with higher blood pressure.

Hemolysis assay was carried out as follows. Rabbit whole blood erythrocytes were collected by centrifugation (200×g, 10 minutes) and diluted to 5% (v/v) with physiological saline. 50µL of PLA-TA-Tro-Ga (DMSO), PLA-TA-Tro (DMSO), DMSO, physiological saline (negative control), and deionized water (positive control) were added separately to 1mL of erythrocyte solution and incubated at 37°C for 24 hours. After centrifugation of the erythrocyte suspensions (500×g, 15 minutes), the supernatant was transferred to a 96 well plate and the absorbance was measured using a microplate reader at 540 nm. The hemolysis rate was calculated as follows. Hemolysis rate (%) = (As-An)/ (Ad-An) × 100% (As, An and Ad are the absorbance of the sample, physiological saline and deionized water, respectively). To ensure the reliability of the data, the experiment was repeated three times for each group. The results are shown in Figure 6, showing thst the hemolysis rate of PLA-TA-Tro-Ga (DMSO) is less than 5%, which meets the requirements for the biomaterials.

Cytotoxicity assay was carried out as follows. L929 cells were added into 12-well plates at 25000 cells per well and incubated in an incubator at 37°C for 12 hours. Then, 50mg of PLA-TA-Tro-Ga (Gel), PLA-TA-Tro (Gel), and Ga were added separately into the wells containing the cells, and the wells without any material added were used as the positive control. After 24 hours of incubation, cell viability was assessed by live/dead cell staining method. To ensure the reliability of the data, the experiment was repeated three times for each group. The results are shown in Figure 6, the cell viability is greater than 98% in all groups and the cells show a normal spindle shape in spreading. This result indicates that the gel is not cytotoxic and has good cell biocompatibility.

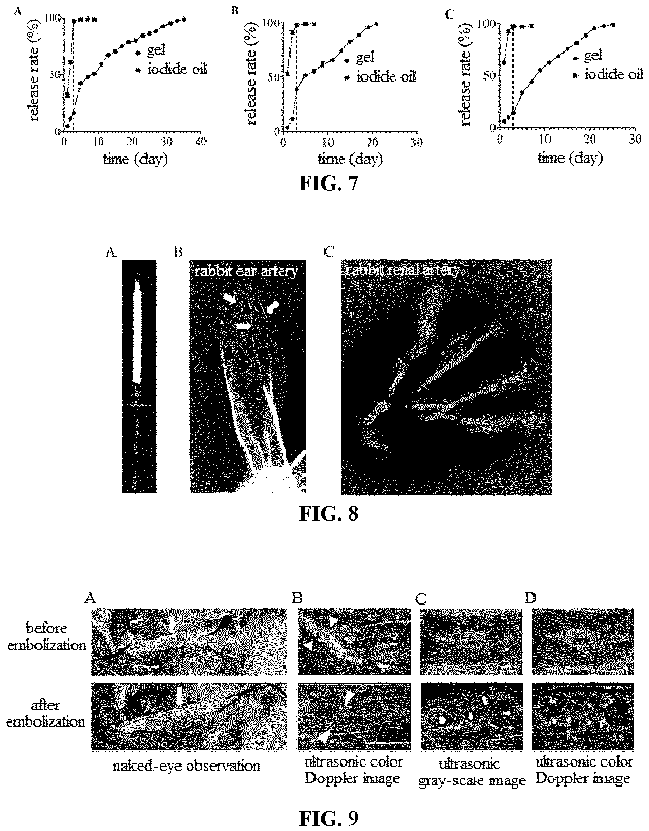

Adriamycin sustained release testing was carried out as follows. The UV-Vis spectrum of the adriamycin solution was measured and the wavelength (480nm) corresponding to the maximum absorbance was used as the characteristic wavelength. Adriamycin solutions of different concentrations were formulated, their absorbance at 480nm was measured and the standard curve of absorbance versus concentration was plotted. 10mg of Adriamycin was dissolved in 2mL of PLA-TA-Tro-Ga (DMSO) and incubated at 37°C for 24 hours. This solution was then injected into 20mL of phosphate buffered salt (PBS) solution. The absorbance of the supernatant (200µL) was measured at 480nm at the set time point to calculate the concentration of adriamycin according the standard curve of absorbance versus concentration. Iodide oil loaded with adriamycin was used as a control. The results, as shown in Figure 7A, show that PLA-TA-Tro-Ga(Gel) can achieve the sustained-release of adriamycin, reaching nearly 100% release on day 35, while iodide oil reached nearly 100% release on day 3, indicating that the vascular embolic agent of the present disclosure can be loaded with chemotherapeutic drugs such as adriamycin and has a good sustained-release effect.

Sorafenib release testing was carried out as follows. The UV-Vis spectrum of the sorafenib solution was measured and the wavelength (265nm) corresponding to the maximum absorbance was used as its characteristic wavelength. Sorafenib solutions of different concentrations were formulated, their absorbance at 265nm was measured and the standard curve of absorbance versus concentration was plotted. Then 200mg of sorafenib was dissolved in 2mL of PLA-TA-Tro-Ga (DMSO) and incubated at 37°C for 24 hours. After which this solution was injected into 20mL of phosphate buffered salt (PBS) solution. The absorbance of the supernatant (200µL) was measured at 265nm at the set time point to calculate the concentration of sorafenib according the standard curve of absorbance versus concentration. Iodide oil loaded with sorafenib was used as a control. The results, as shown in Figure 7B, show that PLA-TA-Tro-Ga(Gel) can achieve the sustained-release of sorafenib, reaching nearly 100% release on day 21, while iodide oil reached nearly 100% release on day 3, indicating that the vascular embolic agent of the present disclosure has a good sustained-release effect for targeting drugs such as sorafenib.

Atelizumab release testing was carried out as follows. The concentration of the monoclonal antibody was detected using an enzyme-linked immunosorbent assay (ELISA) kit (R&D, DY1086). The absorbance of the standard in the kit at 450 nm was detected by using a microplate reader, and the standard curve of absorbance versus concentration was plotted. 0.1 µg of programmed cell death protein 1 (PD-1) was added into a 96-well plate. 60mg of atelizumab was dissolved in 2mL of PLA-TA-Tro-Ga (DMSO) and incubated at 37°C for 24 hours. After which, the mixture containing atelizumab was injected into 20mL of phosphate buffered saline (PBS) solution. The supernatant (200µL) was diluted 1000-fold with PBS at the set time point, and 500µL of this diluted solution was added into the 96-well plate containing PD-1 protein. Then, peroxidase affinipure goat anti-human IgG secondary antibody was added and the 3,3',5,5'-tetramethylbenzidine (TMB) was used for staining. Finally, the absorbance was read at 450 nm using a microplate reader, and the concentration of atilizumab was calculated from the standard curve of absorbance versus concentration. Iodide oil loaded with atilizumab was used as a control. The results, as shown in Figure 7C, show that PLA-TA-Tro-Ga (Gel) can achieve the sustained-release of atilizumab, reaching nearly 100% release on day 25, while iodide oil only reached nearly 100% release on day 3, indicating that the vascular embolic of the present disclosure can load immune inhibitors such as atilizumab with a good sustained drug release property.

In-vitro X-ray imaging was carried out as follows. 1mL of PLA-TA-Tro-Ga (DMSO) was drawn into a 1mL syringe for X-ray imaging. As shown in Figure 8A, PLA-TA-Tro-Ga (DMSO) shows a homogeneous and high density image without imaging artifacts under X-ray.

In-vivo X-ray imaging was carried out as follows. 1mL and 1.5mL of PLA-TA-Tro-Ga (DMSO) were injected into the ear artery and renal artery of rabbits, respectively, using a syringe, and PLA-TA-Tro-Ga (DMSO) filling arteries and branching blood vessels could be clearly seen without imaging artifacts under X-ray (Figure 8B, 8C).

Embolization of the rabbit renal artery was carried out as follows. After general anaesthesia, the rabbit was fixed, and the towel was disinfected. An abdominal midline incision was made through skin, muscle and fascia layer by layer to separate the renal artery by blunt dissection. The renal artery was imaged using an ultrasonic color Doppler for observation. 1.5mL of PLA-TA-Tro-Ga (DMSO) was then injected into the renal artery. After 1 minute, the needle was withdrawn without gel adhesion to the needle and without bleeding from the puncture site, indicating that this liquid embolic agent does not adhere to the catheter. Next, the embolization site was imaged using an ultrasonic color Doppler for observation. As shown in Figure 9, after embolization, the blood flow signals of renal artery disappeared, and there were multiple scattered hypoechoic areas in the renal parenchyma. This result indicates that PLA-TA-Tro-Ga (DMSO) can embolize successfully the renal artery of rabbits after gelation.

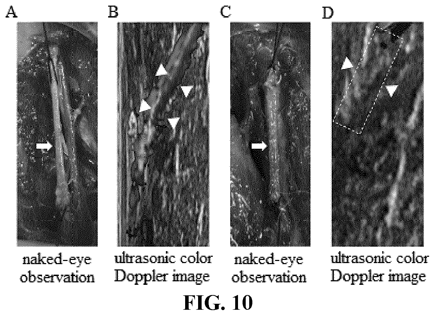

Embolization of the rabbit femoral artery was carried out as follows. After general anaesthesia, the rabbit was fixed, and the towel was disinfected. A left inguinal incision was made through skin and muscle to separate the femoral artery by blunt dissection. The femoral artery was imaged using an ultrasonic color Doppler for observation. 1mL of PLA-TA-Tro-Ga (DMSO) was then injected into the femoral artery. After 1 minute, the needle was withdrawn without gel adhesion to the needle and without bleeding from the puncture site, indicating that this liquid embolic agent did not adhere to the catheter. Next, the embolization site was imaged using an ultrasonic color Doppler for observation. As shown in Figure 10, the blood flow signals from femoral artery disappeared after embolization. This result indicates that PLA-TA-Tro-Ga (DMSO) can successfully embolize the femoral artery of rabbits after gelation.

### Example 3

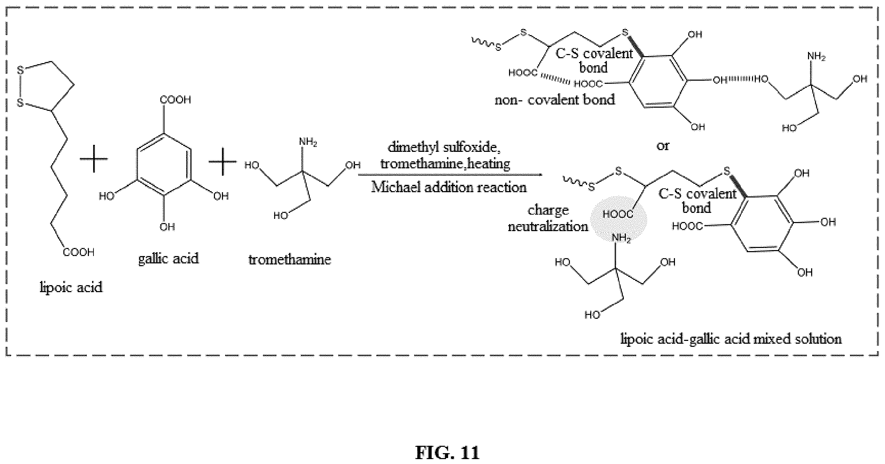

Provided is an example of the present disclosure, and the vascular embolic agent in this example was prepared as follows.
(1) 1.2g of lipoic acid, 0.8g of gallic acid and 0.6g of tromethamine powder were dissolved in 0.6g of DMSO to obtain a mixed solution A;
(2) the mixed solution A was sealed and placed under 90°C for reaction for 10 hours to obtain a mixed solution B; and
(3) the mixed solution B was cooled to room temperature, 2.5g of DMSO was added to for dilution, finally, 1.5g of gallium-indium-tin alloy (the contrast agent) was added to obtain the vascular embolic agent. The resulting vascular embolic agent had a solidification time of 10 minutes, a viscosity of 89mPa.s, and a storage modulus of 470kPa. The schematic diagram of the preparation process of the vascular embolic agent in this example is shown in Figure 11.

### Example 4

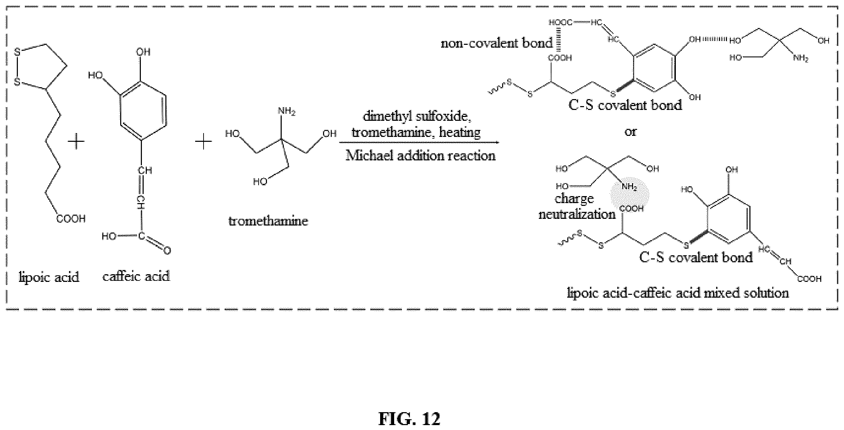

Provided is an example of the present disclosure, and the vascular embolic agent in this example was prepared as follows.
(1) 1.2g of lipoic acid, 0.5g of caffeic acid and 0.6g of tromethamine powder were dissolved in 0.6g of DMSO to obtain a mixed solution A;
(2) the mixed solution A was sealed and placed under 90°C for reaction for 10 hours to obtain a mixed solution B; and
(3) the mixed solution B was cooled to room temperature, 2.5g of DMSO was added for dilution, finally, 1.0g of gallium-indium-tin alloy as contrast agent was added to obtain the vascular embolic agent. The resulting vascular embolic agent had a solidification time of 9 minutes, a viscosity of 92mPa.s, and a storage modulus of 480kPa. The schematic diagram of the preparation process of the vascular embolic agent in this example is shown in Figure 12.

### Example 5

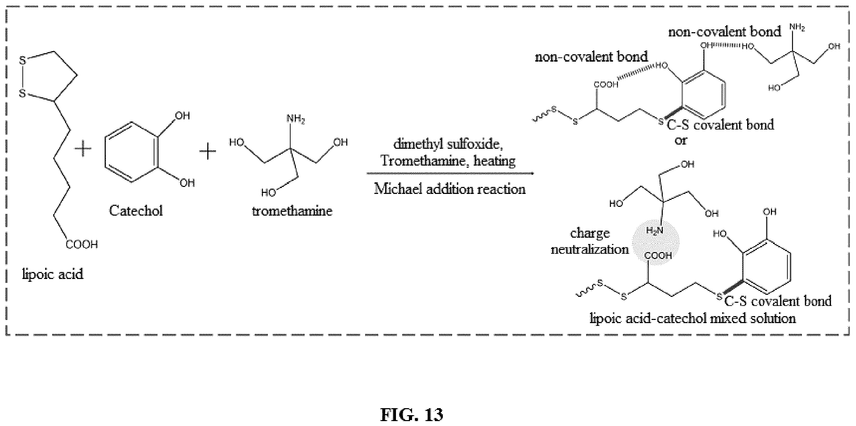

Provided is an example of the present disclosure, and the vascular embolic agent in this example was prepared as follows.
(1) 1.2g of lipoic acid, 0.8g of catechol and 0.6g of tromethamine powder were dissolved in 0.6g of DMSO to obtain a mixed solution A;
(2) the mixed solution A was sealed and placed under 90°C for reaction for 10 hours to obtain a mixed solution B; and
(3) the mixed solution B was cooled to room temperature, 1.5g of DMSO was added for dilution, finally, 1.2g of gallium-indium-tin alloy as contrast agent was added to obtain the vascular embolic agent. The resulting vascular embolic agent had a solidification time of 11 minutes, a viscosity of 80mPa.s, and a storage modulus of 465kPa. The schematic diagram of the preparation process of the vascular embolic agent in this example is shown in Figure 13.

### Example 6

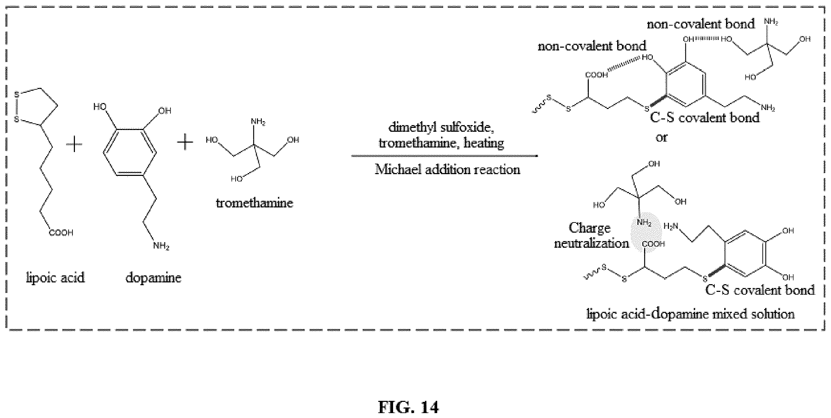

Provided is an example of the present disclosure, and the vascular embolic agent in this example was prepared as follows.
(1) 1.2g of lipoic acid, 1.0g of dopamine and 0.6g of tromethamine powder were dissolved in 0.6g of DMSO to obtain a mixed solution A;
(2) the mixed solution A was sealed and placed under 90°C for reaction for 10 hours to obtain a mixed solution B; and
(3) the mixed solution B was cooled to room temperature, 2.5g of DMSO was added for dilution, finally, 1.6g of gallium-indium-tin alloy as contrast agent was added to obtain the vascular embolic agent. The resulting vascular embolic agent had a solidification time of 9 minutes, a viscosity of 82mPa.s, and a storage modulus of 493kPa. The schematic diagram of the preparation process of the vascular embolic agent in this example is shown in Figure 14.

### Example 7

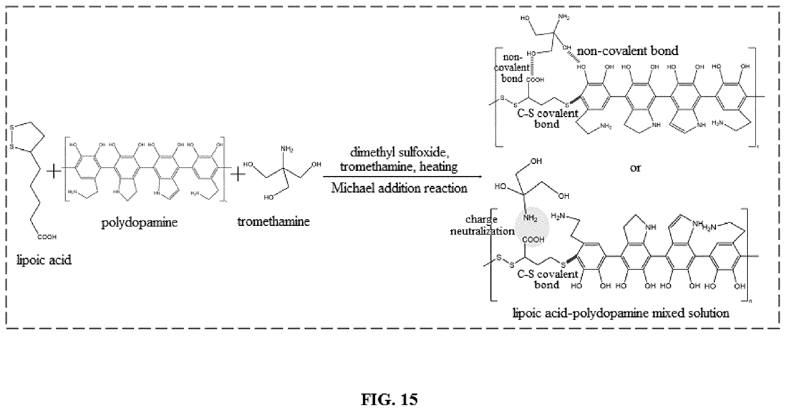

Provided is an example of the present disclosure, and the vascular embolic agent in this example was prepared as follows.
(1) 1.2g of lipoic acid, 0.2g of polydopamine and 0.6g of tromethamine powder were dissolved in 0.4g of DMSO to obtain a mixed solution A;
(2) the mixed solution A was sealed and placed under 90°C for reaction for 10 hours to obtain a mixed solution B; and
(3) the mixed solution B was cooled to room temperature, 1.5g of DMSO was added for dilution, finally, 1.2g of gallium-indium-tin alloy as contrast agent was added to obtain the vascular embolic agent. The resulting vascular embolic agent had a solidification time of 12 minutes, a viscosity of 77mPa.s, and a storage modulus of 520kPa. The schematic diagram of the preparation process of the vascular embolic agent in this example is shown in Figure 15.

### Example 8

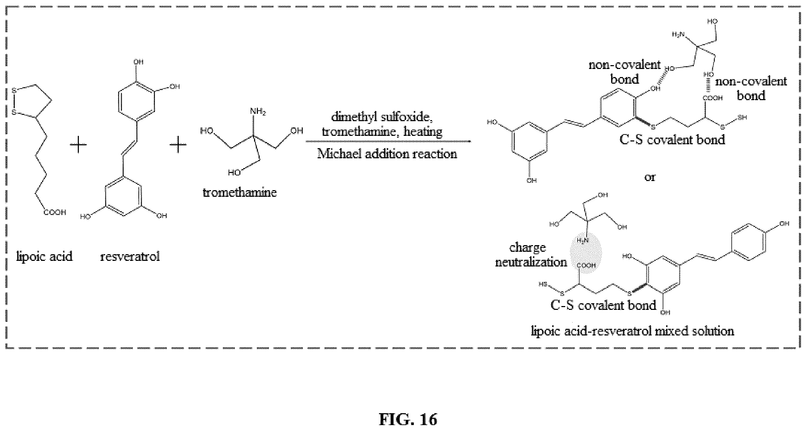

Provided is an example of the present disclosure, and the vascular embolic agent in this example was prepared as follows.
(1) 1.2g of lipoic acid, 0.2g of resveratrol and 0.6g of tromethamine powder were dissolved in 0.4g of DMSO to obtain a mixed solution A;
(2) the mixed solution A was sealed and placed under 90°C for reaction for 10 hours to obtain a mixed solution B; and
(3) the mixed solution B was cooled to room temperature, 1.5g of DMSO was added for dilution, finally, 1.0g of gallium-indium-tin alloy as contrast agent was added to obtain the vascular embolic agent. The resulting vascular embolic agent had a solidification time of 10 minutes, a viscosity of 86mPa.s, and a storage modulus of 489kPa. The schematic diagram of the preparation process of the vascular embolic agent in this example is shown in Figure 16.

### Example 9

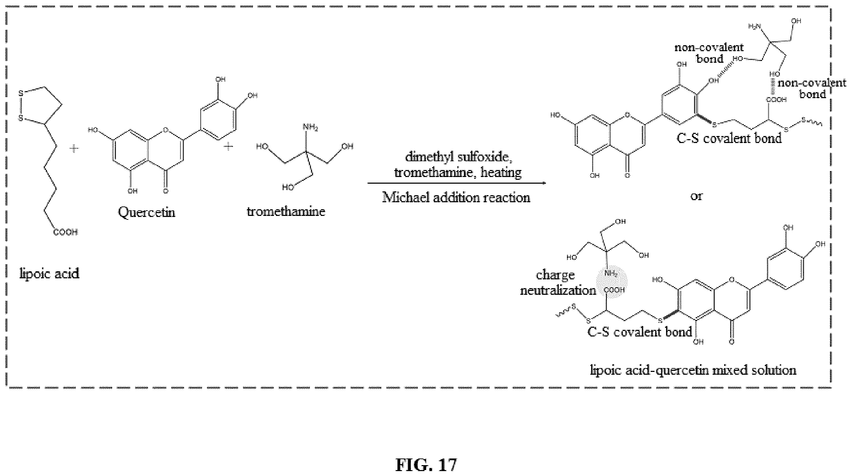

Provided is an example of the present disclosure, and the vascular embolic agent in this example was prepared as follows.
(1) 1.2g of lipoic acid, 0.6g of quercetin and 0.6g of tromethamine powder were dissolved in 0.5g of DMSO to obtain a mixed solution A;
(2) the mixed solution A was sealed and placed under 90°C for reaction for 10 hours to obtain a mixed solution B; and
(3) the mixed solution B was cooled to room temperature, 2.0g of DMSO was added for dilution, finally, 1.2g of gallium-indium-tin alloy as contrast agent was added to obtain the vascular embolic agent. The resulting vascular embolic agent had a solidification time of 11 minutes, a viscosity of 76mPa.s, and a storage modulus of 490kPa. The schematic diagram of the preparation process of the vascular embolic agent in this example is shown in Figure 17.

### Example 10

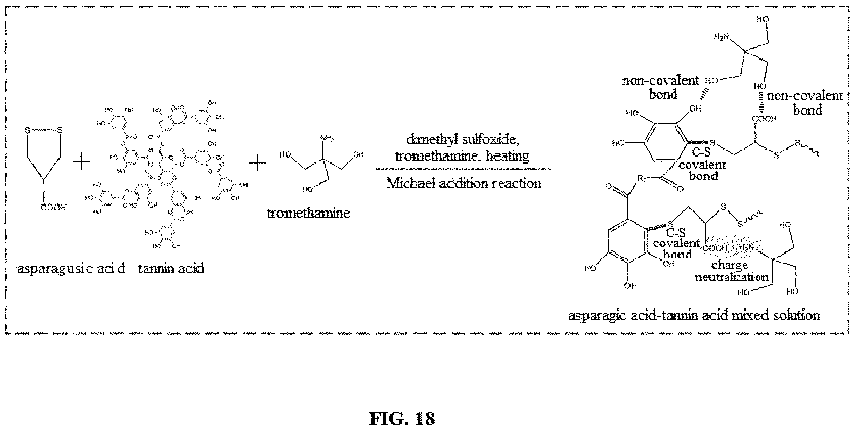

Provided is an example of the present disclosure, and the vascular embolic agent in this example was prepared as follows.
(1) 1.4g of asparagusic acid, 0.1g of tannic acid and 0.5g of tromethamine powder were dissolved in 0.4g of DMSO to obtain a mixed solution A;
(2) the mixed solution A was sealed and placed under 90°C for reaction for 10 hours to obtain a mixed solution B; and
(3) the mixed solution B was cooled to room temperature, 2.0g of DMSO was added for dilution, finally, 1.0g of gallium-indium-tin alloy as contrast agent was added to obtain the vascular embolic agent. The resulting vascular embolic agent had a solidification time of 9 minutes, a viscosity of 80mPa.s, and a storage modulus of 477kPa. The schematic diagram of the preparation process of the vascular embolic agent in this example is shown in Figure 18.

### Example 11

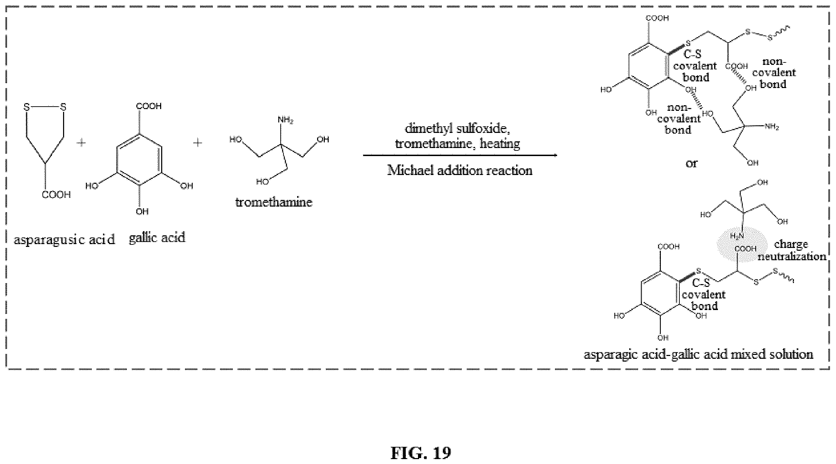

Provided is an example of the present disclosure, and the vascular embolic agent in this example was prepared as follows.
(1) 1.2g of asparagusic acid, 1.0g of gallic acid and 0.5g of tromethamine powder were dissolved in 0.6g of DMSO to obtain a mixed solution A;
(2) the mixed solution A was sealed and placed under 90°C for reaction for 10 hours to obtain a mixed solution B; and
(3) the mixed solution B was cooled to room temperature, 2.0g of DMSO was added for dilution, finally, 1.6g of gallium-indium-tin alloy as contrast agent was added to obtain the vascular embolic agent. The resulting vascular embolic agent had a solidification time of 8 minutes, a viscosity of 80mPa.s, and a storage modulus of 466kPa. The schematic diagram of the preparation process of the vascular embolic agent in this example is shown in Figure 19.

### Example 12

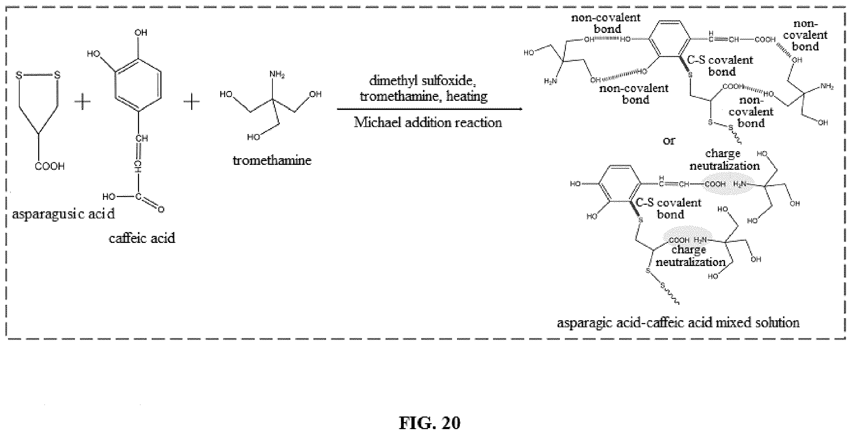

Provided is an example of the present disclosure, and the vascular embolic agent in this example was prepared as follows.
(1) 1.2g of asparagusic acid, 1.0g of caffeic acid and 0.5g of tromethamine powder were dissolved in 0.5g of DMSO to obtain a mixed solution A;
(2) the mixed solution A was sealed and placed under 90°C for reaction for 10 hours to obtain a mixed solution B; and
(3) the mixed solution B was cooled to room temperature, 2.5g of DMSO was added for dilution, finally, 1.8g of gallium-indium-tin alloy as contrast agent was added to obtain the vascular embolic agent. The resulting vascular embolic agent had a solidification time of 9 minutes, a viscosity of 77mPa.s, and a storage modulus of 482kPa. The schematic diagram of the preparation process of the vascular embolic agent in this example is shown in Figure 20.

### Example 13

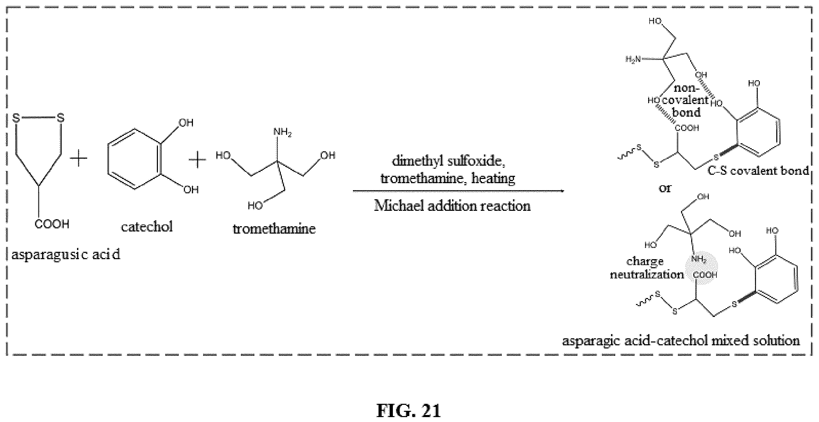

Provided is an example of the present disclosure, and the vascular embolic agent in this example was prepared as follows.
(1) 1.4g of asparagusic acid, 1.0g of catechol and 0.5g of tromethamine powder were dissolved in 0.6g of DMSO to obtain a mixed solution A;
(2) the mixed solution A was sealed and placed under 90°C for reaction for 10 hours to obtain a mixed solution B; and
(3) the mixed solution B was cooled to room temperature, 3.0g of DMSO was added for dilution, finally, 1.8g of gallium-indium-tin alloy as contrast agent was added to obtain the vascular embolic agent. The resulting vascular embolic agent had a solidification time of 10 minutes, a viscosity of 86mPa.s, and a storage modulus of 489kPa. The schematic diagram of the preparation process of the vascular embolic agent in this example is shown in Figure 21.

### Example 14

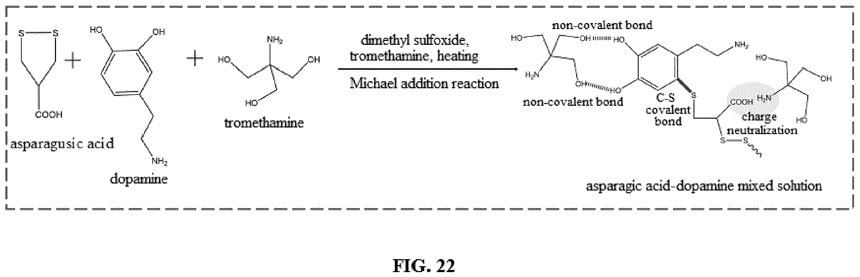

Provided is an example of the present disclosure, and the vascular embolic agent in this example was prepared as follows.
(1) 1.4g of asparagusic acid, 0.6g of dopamine and 0.5g of tromethamine powder were dissolved in 0.5g of DMSO to obtain a mixed solution A;
(2) the mixed solution A was sealed and placed under 90°C for reaction for 10 hours to obtain a mixed solution B; and
(3) the mixed solution B was cooled to room temperature, 2.5g of DMSO was added for dilution, finally, 1.2g of gallium-indium-tin alloy as contrast agent was added to obtain the vascular embolic agent. The resulting vascular embolic agent had a solidification time of 12 minutes, a viscosity of 65mPa.s, and a storage modulus of 412kPa. The schematic diagram of the preparation process of the vascular embolic agent in this example is shown in Figure 22.

### Example 15

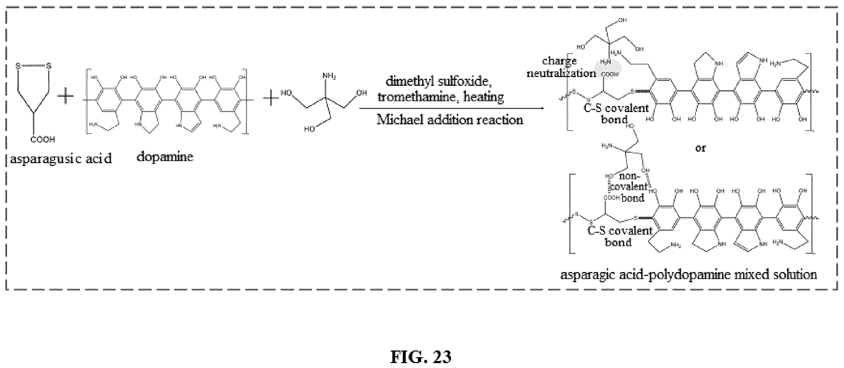

Provided is an example of the present disclosure, and the vascular embolic agent in this example was prepared as follows.
(1) 1.4g of asparagusic acid, 0.1g of polydopamine and 0.5g of tromethamine powder were dissolved in 0.5g of DMSO to obtain a mixed solution A;
(2) the mixed solution A was sealed and placed under 90°C for reaction for 10 hours to obtain a mixed solution B; and
(3) the mixed solution B was cooled to room temperature, 2.0g of DMSO was added for dilution, finally, 1.0g of gallium-indium-tin alloy as contrast agent was added to obtain the vascular embolic agent. The resulting vascular embolic agent had a solidification time of 9 minutes, a viscosity of 78mPa.s, and a storage modulus of 482kPa. The schematic diagram of the preparation process of the vascular embolic agent in this example is shown in Figure 23.

### Example 16

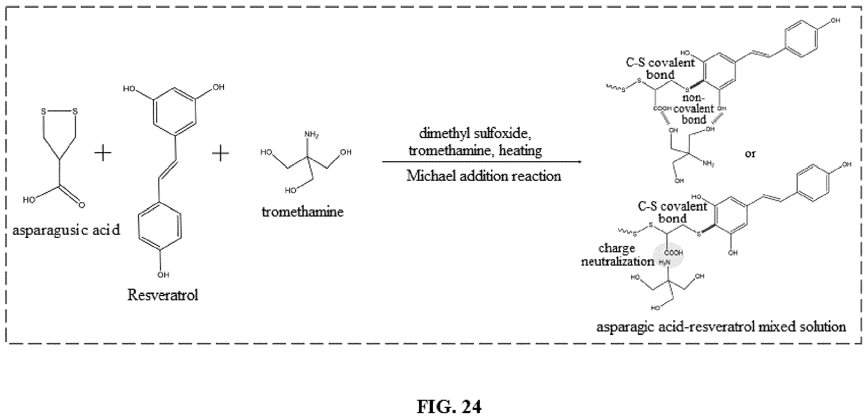

Provided is an example of the present disclosure, and the vascular embolic agent in this example was prepared as follows.
(1) 1.4g of asparagusic acid, 0.5g of resveratrol and 0.5g of tromethamine powder were dissolved in 0.5g of DMSO to obtain a mixed solution A;
(2) the mixed solution A was sealed and placed under 90°C for reaction for 10 hours to obtain a mixed solution B; and
(3) the mixed solution B was cooled to room temperature, 2.5g of DMSO was added for dilution, finally, 1.2g of gallium-indium-tin alloy as contrast agent was added to obtain the vascular embolic agent. The resulting vascular embolic agent had a solidification time of 12 minutes, a viscosity of 65mPa.s, and a storage modulus of 412kPa. The schematic diagram of the preparation process of the vascular embolic agent in this example is shown in Figure 24.

### Example 17

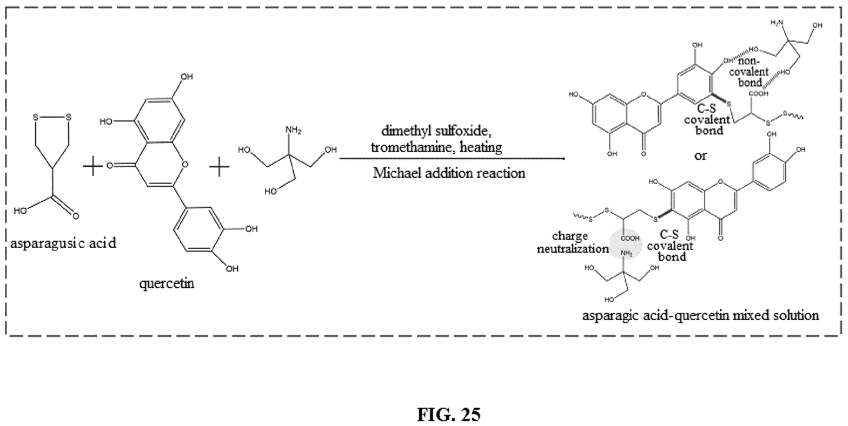

Provided is an example of the present disclosure, and the vascular embolic agent in this example was prepared as follows.
(1) 1.4g of asparagusic acid, 0.3g of quercetin and 0.5g of tromethamine powder were dissolved in 0.4g of DMSO to obtain a mixed solution A;
(2) the mixed solution A was sealed and placed under 90°C for reaction for 10 hours to obtain a mixed solution B; and
(3) the mixed solution B was cooled to room temperature, 2.0g of DMSO was added for dilution, finally, 1.0g of gallium-indium-tin alloy as contrast agent was added to obtain the vascular embolic agent. The resulting vascular embolic agent had a solidification time of 10 minutes, a viscosity of 88mPa.s, and a storage modulus of 378kPa. The schematic diagram of the preparation process of the vascular embolic agent in this example is shown in Figure 25. Finally, it should be noted that, the above examples are only used to illustrate the embodiments of the present application rather than limit the protection scope of the present application. Although the present application has been illustrated in detail with reference to the preferred examples, it should be understood by those skilled in the art that, modifications or equivalent replacements may be made to the embodiments of the present application without departing from the essence and scope of the present application.

## Claims

1. A method for preparing a vascular embolic agent, comprising
step (1), dissolving a 1,2-dithiolane compound, a polyphenol compound and an alkaloid in an organic solvent A to obtain a mixed solution A;
step (2), sealing the mixed solution A, conducting a reaction in an environment of 70°C or above for 5 to 12 hours to obtain a mixed solution B; and
step (3), cooling the mixed solution B to room temperature, and adding an organic solvent B into the mixed solution B for dilution to obtain the vascular embolic agent.

2. The method according to claim 1, wherein the organic solvent A and the organic solvent B are each selected from the group consisting of dimethyl sulfoxide, ethanol, and a mixture thereof, and the organic solvent A and the organic solvent B are the same or different.

3. The method according to claim 1, wherein in step (1), a mass ratio of the 1,2-dithiolane compound, the polyphenol compound, and the alkaloid is (0.05-1):(0.0001-1):(0.01-1), a mass ratio of the organic solvent A to the mixed solution A is (2-4):10; and in step (3), a mass ratio of the organic solvent B to the vascular embolic agent is (1-5.8):10.

4. The method according to claim 3, wherein in step (1), the mass ratio of the 1,2-dithiolane compound, the polyphenol compound, and the alkaloid is 0.6:0.05:0.3.

5. The method according to claim 1, wherein the 1,2-dithiolane compound is at least one of lipoic acid, asparagusic acid, dithiolopyrrolone antibiotic, and kottamide E; the polyphenol compound is at least one of tannic acid, gallic acid, caffeic acid, catechol, dopamine, polydopamine, resveratrol, quercetin, curcumin, chlorogenic acid, isoflavone, anthocyanin, cocoa polyphenol, limocitrin, catechin, and rutin; the alkaloid is at least one of tromethamine, ephedrine, leonurine and cinchona.

6. The method according to claim 5, wherein the 1,2-dithiolane compound is lipoic acid, the polyphenol compound is tannic acid, the alkaloid is tromethamine, and the organic solvent A and the organic solvent B are dimethyl sulfoxide.

7. The method according to claim 1, wherein in step (3), after dilution, a contrast agent is added in an amount of 10wt%-80wt% by weight of the vascular embolic agent, and the contrast agent is a liquid metal having a melting point of less than 35°C or tantalum microparticle.

8. The method according to claim 7, wherein the contrast agent is added in an amount of 30wt% of the vascular embolic agent, and the contrast agent is a gallium-indium alloy.

9. A vascular embolic agent, wherein the vascular embolic agent is prepared by the method according to any one of claims 1 to 8.

10. Use of the vascular embolic agent according to claim 9 in the manufacture of a medicament carrier.
